(19) Europäisches Patentamt
European Patent Office
Office européen des brevets

(11) Publication number: **0 321 990 B1**

(12) **EUROPEAN PATENT SPECIFICATION**

(45) Date of publication of patent specification: **14.04.93**

(51) Int. Cl.[5]: **C07C 43/12**, C07C 22/00, C07C 41/06, C07C 17/26

(21) Application number: **88121539.6**

(22) Date of filing: **22.12.88**

(54) **New chlorotrifluoroethylene telomers and process for preparing them.**

(30) Priority: **23.12.87 IT 2317987**

(43) Date of publication of application:
**28.06.89 Bulletin 89/26**

(45) Publication of the grant of the patent:
**14.04.93 Bulletin 93/15**

(84) Designated Contracting States:
**BE DE FR GB IT NL**

(56) References cited:
**FR-A- 1 206 546**
**GB-A- 927 403**
**GB-A- 2 148 286**

**CHEMICAL ABSTRACTS, vol. 104, no. 13, 31st March 1986, page 667, abstract no. 109007z, Columbus, Ohio, US; & JP-A-60 184 032**

(73) Proprietor: **AUSIMONT S.p.A.**
**Foro Buonaparte, 31**
**I-20121 Milano(IT)**

(72) Inventor: **Marraccini, Antonio, Dr.**
**3, corso Riviera**
**I-28040 Dormelletto Novara(IT)**
Inventor: **Perego, Gabriele, Dr.**
**3, via Fossigalli**
**I-10015 Ivrea Torino(IT)**
Inventor: **Guastalla, Giovanni, Dr.**
**63/A, via Fara**
**I-28100 Novara(IT)**

(74) Representative: **Barz, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40 (DE)**

**EP 0 321 990 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new telomers of chlorotrifluoroethylene. Particularly, it relates to new telomers prepared by reaction of chlorotrifluoroethylene with perhalofluoroxy compounds.

Chlorotrifluoroethylene telomers are utilized in particular as hydraulic fluids. In general, they are prepared by reacting chlorotrifluoroethylene with chlorinated telogens, such as e.g. $CCl_4$ and $SO_2Cl_2$. The telomers so obtained contain polychlorinated end groups, for example $-CFCl_2$ and $-CCl_3$ which, in applications where high temperatures or oxidizing media are used, can be hydrolysed or oxidized and result in corrosive acid groups.

Due to the above drawbacks, the telomers so obtained require a stabilizing fluorination treatment for the conversion of the polychlorinated end groups to groups having a higher fluorine content. This treatment is complex and expensive.

GB-A-2,148,286 describes a process for the production of telomers of formula $CF_3O(CFClCF_2)_nX$ wherein n ranges from 1 to 10 and X represents F or $OCF_3$, which comprises reacting chlorotrifluoroethylene with fluoroxytrifluoromethane or bisfluoroxydifluoromethane.

JP-A-60/184,032 (Chem. Abstr. Vol. 104, No. 13, March 31,1986, Abstr. no. 109007z) describes chlorotrifluoroethylene oligomers of formula $R(C_2F_3Cl)_nR^1$ wherein R = fluoroalkyl, $R^1$ = F or fluoroalkyl and n = 1-20, prepared by telomerization of $C_2F_3Cl$ with RI and subsequent fluorination or coupling reaction.

GB-A-927,403 discloses, among others, a composition containing compounds of formulae $CF_3CFCl-(CF_2CFCl)_nF$ and $CF_3CCl_2(CF_2CFCl)_nF$ wherein n = 4 to 6.

FR-A-1,206,546 is concerned with compounds of formula $X(CF_2CFCl)_nY$ wherein X and Y preferably represent F, Cl, $CF_3$, $CF_2Cl$ or $CCl_2F$ and n ranges from 3 to 15.

Furthermore, US-A- 4,577,044 describes chlorotrifluoroethylene telomers obtained by reaction of $CF_2CFCl$ with $CF_3OF$. The following telomeric species were obtained: $F(CF_2-CFCl)_nF$, $CF_3O-(CF_2CFCl)_n-F$ and $CF_3O-(CF_2CFCl)_n-OCF_3$, in which n usually ranges from 1 to 10 and telomeric units ($CF_2$-CFCl) are randomly distributed: i.e. they can be linked to one another both in head-to-head and head-to-tail fashion.

Since said telomers have a low degree of telomerization, it is assumed that the nature of the end groups and the type of bond (namely oxygen-carbon or carbon-carbon) by which these groups are bound to the monomeric units, may exert a considerable influence on the physical and chemical properties of the telomers, due, in particular, to different binding forces, different sizes of the groups and differences in the steric environment of said groups and in the flexibility of the type of bond (oxygen-carbon or carbon-carbon).

Consequently, the telomers of chlorotrifluoroethylene with $CF_3OF$ cannot exhibit a large variety of properties as they possess only the $CF_3O$- and F- end groups. On the other hand, it is well known that an optimum property for a particular application of the chlorotrifluoroethylene telomers may be a handicap for another application.

Thus, it is an object of the present invention to provide a process for the production of chlorotrifluoroethylene telomers with inert end groups both of the perhaloalkyl type (which consequently bind to the monomeric units by means of a carbon-carbon bond) and of the perhaloalkoxy type, i.e. of the ether type (which bind to the monomeric units by means of an oxygen-carbon bond).

Another object is that of providing a process which allows to vary, over a wide range, both distribution and nature of the end groups which are present in the telomers.

A further object is that of providing a process which allows to vary, over a wide range, the F/Cl ratio in the telomers. This embodiment has two important aspects. On the one hand, a higher F/Cl ratio, achieved by means of ether and non-ether perfluorinated end groups, leads to a modification of the physical and chemical properties of the telomers, which makes it possible, for example, to obtain lower pour point temperatures, lower heat of combustion values, a lower surface tension and a different variation of the viscosity as a function of temperature. On the other hand, for certain uses, a low F/Cl ratio, achieved through end groups containing Cl and F atoms, of the ether and non-ether type, results in special properties such as, for example, a decrease in the compressibility.

A still further object of the present invention is to provide a process which allows, within certain limits, to control the telomerization reaction so as to obtain telomeric species having a low telomerization degree or, on the contrary, a higher telomerization degree.

It is therefore apparent that the present invention provides a very flexible process capable of yielding a very broad range of products, with different physical and chemical characteristics, which makes it possible to meet various applicative requirements.

These and still other objects are achieved by the process of the present invention for preparing chlorotrifluoroethylene telomers. This process is characterized in that chlorotrifluoroethylene is reacted, at a temperature ranging from -100 to +40°C, with a perhalofluoroxy compound of formula $R_x$-$CF_2OF$, wherein $R_x$ is a perhalogenated alkyl radical, a perhaloalkylmonoether radical or a perhaloalkylpolyether radical, either linear or branched, having from 1 to 10 carbon atoms and containing fluorine atoms or fluorine and chlorine atoms, but being free of vicinal chlorine atoms and -$CCl_3$ groups.

It is assumed that the reaction mechanism involves in part the homolytic rupture of the O-F bond of the fluoroxy compound according to the equation:

$R_xCF_2$-OF → $R_x$-$CF_2$-$O^{\cdot}$ + $F^{\cdot}$

wherefore radicals $R_x$-$CF_2$-$O^{\cdot}$ and $F^{\cdot}$ can act as telomerization starters and terminators. It is likewise assumed that the above radicald can also undergo further fragmentation and re-arrangement reactions with formation of other radicals which, in turn, can act as telomerization starters and terminators. In the case of radicals $R_x$ containing Cl atoms, it is furthermore supposed that one or more chlorine atoms can be substituted by fluorine atoms.

When starting from a perfluorofluoroxy compound, $R^1$ radicals derived from $R_x$ form, in which $R_x$ having at least two carbon atoms has lost one or more carbon atoms and/or $R_x$ having at least three carbon atoms has undergone a re-arrangement. More frequently, radical $R^1$ is a radical $R^2$ which contains a lower number of carbon atoms with respect to $R_x$. Thus, when $R_x$ contains from 2 to 10 carbon atoms, $R^2$ contains from 1 to 9 carbon atoms. Starting from a perhalofluoroxy compound which contains chlorine, radicals $R^3$ derived from $R_x$ are formed, in which $R_x$ having at least two carbon atoms has lost one or more carbon atoms and/or $R_x$ having at least 3 carbon atoms has undergone a re-arrangement and one or more chlorine atoms of $R_x$ have been substituted by fluorine atoms.

More commonly, radical $R^3$ is a radical $R^4$ which contains a lower number of carbon atoms with respect to $R_x$ (i.e. when $R_x$ contains from 2 to 10 carbon atoms, $R^4$ contains from 1 to 9 carbon atoms) and in which one or more chlorine atoms have been substituted by fluorine atoms.

It has been found that the telomerization product consists of a mixture of telomers having different end groups.

When starting from a perfluorofluoroxy compound, the telomers obtained are the following (in each telomer, the monomeric unit $CF_2$-CFCl is schematically represented by the letter M):

$R_x$-$CF_2$-O-$(M)_n$-F (A)

wherein $R_x$ has the meaning defined above and "n" ranges from 2 to 20.

$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$R_x$-O-$(M)_n$-F (F)
$R_x$-O-$(M)_n$-O-$CF_2$-$R_x$ (G)
$R_x$-O-$(M)_n$-$R_x$ (H)
$R_x$-$CF_2$-$(M)_n$-F (I)
$R_x$-$CF_2$-$(M)_n$-O-$CF_2$-$R_x$ (J)
$R_x$-$CF_2$-$(M)_n$-$R_x$ (K)
$R^1$-O-$(M)_n$-$OCF_2$-$R_x$ (L)

wherein $R^1$ is a radical derived from $R_x$, in which $R_x$ having at least two carbon atoms has lost one or more carbon atoms and/or $R_x$ having at least three carbon atoms has undergone a re-arrangement.

$R^1$-O-$(M)_n$-$R_x$ (N)
$R^1$-$(M)_n$-F (O)
$CF_3$-O-$(M)_n$-F (P)
F-$(M)_n$-F (Q).

The telomers from (A) to (E) and from (G) to (O) are new compounds as long as in formulae (C), (I) and (O) $R_x$ and $R^1$, resp. is different from perfluoroalkyl and in formula (D) $R_x$ is different from perhalomethyl

when n ranges from 3 to 15. The telomers (F) are new when $R_x$ is different from $-CF_3$.

When starting from a perhalofluoroxy compound which contains chlorine, the telomers obtained are the following (in each of the telomers, the monomeric unit $CF_2$-CFCl is schematically represented by M: (A), (B), (C), (D), (E), (P) and (Q), indicated above

$R^3$-O-$(M)_n$-$OCF_2$-$R_x$ (R)

wherein $R_x$ and n have the meaning defined above while $R^3$ is a radical derived from $R_x$, in which $R_x$ having at least two carbon atoms has lost one or more carbon atoms and/or $R_x$ having at least three carbon atoms has undergone a re-arrangement and one or more chlorine atoms of $R_x$ have been substituted by fluorine atoms

$R^3$-O-$(M)_m$-$R_x$ (S)
$R^3$-$CF_2$-O-$(M)_n$-F (T)
$R^3$-$CF_2$-O-$(M)_n$-O-$CF_2$-$R_x$ (U)
$R^3$-$CF_x$-O-$(M)_n$-$R_x$ (V)

Telomers (R), (S), (T), (U) and (V) are new products. In the telomer chain $(M)_n$ the monomeric units $CF_2$-CFCl are bonded to each other at random, i.e. they follow one another both in a head-to-head and a head-to-tail fashion.

It has to be borne in mind that in the indicated telomer formulae (M) can be either ($CF_2$-CFCl) or (CFCl-$CF_2$). So, with telomers $R_x$-$CF_2$-O-$(M)_n$-F (A) there are two corresponding series of products represented by the formulae:

1) $R_x$-$CF_2$-O-$(CF_2\text{-}CFCl)_n$-F ($A_1$)

in which the end group $R_x$-$CF_2$-O- is bound to the group $CF_2$, while the end group F is bound to the group CFCl, independently of the linkage of the intermediate monomeric units when n is higher than 2.

2) $R_x$-$CF_2$-O-$(CFCl\text{-}CF_2)_n$-F ($A_2$)

in which the end group $R_x$-$CF_2$-O- is bound to the group CFCl, while the end group F is bound to the group $CF_2$, independently of the linkage of the intermediate monomeric units when n is higher than 2.

In the telomer mixtures obtained from perfluorofluoroxy compounds or from perhalofluoroxy compounds containing chlorine, small amounts of telomers having a formula different from those indicated above can be present.

Examples of $R_x$ are perhalogenated methyl, ethyl, n-propyl, i-propyl, n-butyl, sec.-butyl, i-butyl, tert.-butyl, pentyl, hexyl, heptyl, octyl, nonyl and decyl. Said groups may have e.g. one or two interspersed O atoms, such as e.g. methoxyethyl, ethoxyethyl, ethoxyethoxyethyl etc.

When $R_x$ is a perhaloalkylpolyether radical, it preferably contains two oxygen atoms.

Preferably, the radical $R_x$ contains from 1 to 5 carbon atoms.

As already mentioned herein, when $R_x$ contains chlorine, it is free of vicinal chlorine atoms and $-CCl_3$ groups; furthermore it is preferably free of $-CCl_2-$ groups.

In the preferred products, n generally ranges from 2 to 10.

The telomer mixtures prepared by the process of the present invention are useful in particular as hydraulic fluids and as service fluids (operating fluids).

To carry out the reaction, a gaseous or liquid flow of perhalofluoroxy compound is generally fed to a reactor containing chlorotrifluoroethylene in the liquid state or dissolved in a solvent. When operating under pressure, the reaction can be conducted at a temperature which is higher than the chlorotrifluoroethylene boiling point (-27.9 °C). If the reaction is carried out in solution, a solvent for chlorotrifluoroethylene is used which is inert under the reaction conditions, in particular a chlorofluorohydrocarbon, such as for example 1,2-dichlorotetrafluoroethane, fluorotrichloromethane and dichlorodifluoromethane; usually, the chlorotrifluoroethylene content in the solution ranges from 20 to 80% by weight.

Generally, the fluoroxy compound is fed to the reactor as a gas stream. Preferably, the gaseous fluoroxy compound is diluted with a gas which is inert under the reaction conditions. Examples of inert diluting gases are nitrogen, argon, helium and a gaseous chlorofluorohydrocarbon selected, for example, from 1,2-dichlorotetrafluoroethane and dichlorodifluoromethane. Usually, the fluoroxy compound concentration in the gaseous mixture ranges from 5 to 80% by volume.

If the fluoroxy compound is fed to the reactor in the liquid state, it is subjected to mixing with a liquid which is inert under the reaction conditions, in particular a chlorofluorohydrocarbon, for example 1,2-dichlorotetrafluoroethane, fluorotrichloromethane and dichlorodifluoromethane, or it is carried, in the form of an aerosol, by a gas which is inert under the reaction conditions, for example nitrogen, argon or helium.

It is also possible to feed to the reactor containing a solvent for chlorotrifluoroethylene a gaseous or liquid fluoroxy compound flow, according to one of the feeding methods indicated herein-before, and, separately, a gaseous or liquid chlorotrifluoroethylene flow: in this case, chlorotrifluoroethylene is preferably fed in the liquid state.

The temperature at which the reaction is conducted ranges from -100 to +40 °C. For each fluoroxy compound it is necessary to work at a temperature which is at least equal to the threshold temperature of its reaction with chlorotrifluoroethylene, this temperature varying from telogen to telogen. Furthermore, as will be explained below, the variation of the temperature makes it possible to vary, over a wide range, both distribution and nature of the end groups which are present in the telomers, and to adjust, to a certain extent, the telomerization degree. Preferably, the temperature ranges from -30 to -80 °C.

By means of the process of the present invention it is possible to control the reaction in such way as to obtain prevailingly the telomers of formula:

$R_x$-$CF_2$-O-$(M)_n$-F (A)
$R_x$-$CF_2$-O-$(M)_n$-O-$CF_2$-$R_x$ (B) and
F-$(M)_n$-F (Q).

The result can be achieved by employing conditions which reduce the amount of heat generated in the reaction medium and promote an effective dispersion thereof.

If the telomerization is effected by adduction of a telogen gaseous flow to the chlorotrifluoroethylene, either as a liquid or in solution, this result is obtained by following one or more or all of the following procedures:

1) according to the first procedure, the reaction is conducted in a lower temperature range, provided the temperature is still higher than the threshold temperature of the reaction;

2) another procedure consists in the precooling of the telogen before it is fed to the reactor. In this case, the telogen is employed in the gaseous or in the liquid state: if it is introduced in the liquid state, the feeding is carried out according to the previously described procedure;

3) a further procedure consists in decreasing the telogen flow rate or in increasing its dilution in the inert gas.

Effective stirring in the reactor also helps to obtain the desired result.
Obviously, the lower the temperature of the reaction medium and the more effective the stirring and the lower the precooling temperature of the telogen, the higher is the telogen flow rate which can be used.

By operating according to the above procedures and starting from $CF_3CF_2OF$ it is possible to obtain said telomers (A), (B) and (Q) in amounts which can reach or exceed 90% by weight of the total weight of the reaction products.

Conversely, if higher temperatures of the reaction medium and/or higher telogen flow rates and a less effective stirring are employed, telomers different from (A) and (B) will predominantly be obtained. More precisely, in the case of perfluorinated telogens, for example, the following telomers are predominantly obtained:

$R_x$-$(M)_n$-F (C)
$R_x$-$(M)_n$-$R_x$ (D)
$R_x$-$CF_2$-O-$(M)_n$-$R_x$ (E)
$R_x$O-$(M)_n$-F (F)
$R_x$-$CF_2$-$(M)_n$-F (I)
F-$(M)_n$-F (Q).

In the case of chlorine-containing telogens there are prevailingly obtained, for example, the following telomers.

$R_x$-$(M)_n$-F (C)
$R_x$-$(M)_n$-$R_x$ (D)
$R_x$-$CF_2$-O-$(M)_n$-$R_x$ (E)
F-$(M)_n$-F (Q)

$R^3$-O-$(M)_n$-$R_x$ (S)

When working according to the last mentioned procedures and starting from $CClF_2$-$CF_2OF$, the above telomers are obtained in amounts which can reach or exceed 90% by weight of the total weight of the reaction products.

According to another embodiment of the process of the present invention, using conditions which make it possible to direct the telomerization reaction prevailingly to the desired telomeric species, it is also possible, by reducing the amount of heat evolved in the reaction medium and by promoting an effective dispersion thereof, to direct, within certain limits, the telomerization degree towards low values. This is achieved with low telogen flow rates and with a low temperature of the rection medium and, optionally, by pre-cooling the telogen. Another measure which helps to obtain this result consists in carrying out an effective stirring in the reactor. It is possible to obtain, e.g., up to 80% or more of telomers having a value of "n" ranging from 3 to 6.

By operating under inverse conditions, i.e. with higher telogen flow rates and/or with a higher temperature of the reaction medium and, optionally, with a less intense stirring, the production of telomers having higher values of "n" is promoted.

For each telogen it is possible, therefore, to control the reaction towards the predominant production of telomers containing particular end groups, and to obtain, to a certain extent, a more or less low or a more or less high telomerization degree. It is therefore evident that the process of the present invention allows to obtain a large variety of products capable of meeting various applicative requirements.

Among the fluoroxy compounds utilizable in the process of the present invention, the following can be cited:

fluoroxy pentafluoroethane
1-fluoroxy heptafluoropropane
1-fluoroxy nonafluorobutane
1-fluoroxy 2-chlorotetrafluoroethane
1-fluoroxy 2,2'-dichlorotrifluoroethane
fluoroxy heptafluoroisopropane
fluoroxy nonafluoroisobutane
fluoroxy nonafluoro-ter.butane
1-fluoroxy-2-perfluoro n.propoxy-hexafluoropropane
1-fluoroxy-2-perfluoromethoxy-hexafluoropropane
1-fluoroxy-2-perfluoroethoxy-hexafluoropropane and
1-fluoroxy-3-chlorohexafluoro n.propane.

When $CF_3CF_2OF$ is used as a telogen, the resulting telogen mixture is almost exclusively composed of the following species:

$F(M)_nF$ (Ia)
$F(M)_nOCF_2CF_3$ (IIa)
$CF_3CF_2O(M)_n$-$OCF_2CF_3$ (IIIa)
$CF_3(M)_n$-F (IVa)
$CF_3(M)_n$-$OCF_2CF_3$ (Va)
$CF_3O(M)_n$-$OCF_2CF_3$ (VIa)
$CF_3CF_2$-$(M)_n$-F (VIIa)
$CF_3CF_2(M)_n$-$OCF_2CF_3$ (VIIIa)
$CF_3O(M)_nF$ (IXa)
$CF_3$-$(M)_n$-$CF_3$ (Xa)

By reducing the amount of heat evolved in the reaction medium and by promoting its effective dispersion, according to the procedures specified hereinbefore, species Ia, IIa and IIIa may constitute more than 85% of the obtained telomer mixture.

Conversely, by increasing the amount of heat evolved in the reaction medium, according to the procedures indicated hereinbefore, species Ia and IVa through IXa may constitute more than 90% of the obtained mixture.

When $CF_3$-$CF_2$-$CF_2OF$ is used as the telogen, the obtained telomer mixture is almost exclusively composed of the following species:

$F(M)_nF$ (Ia)

$F(M)_n-OCF_2CF_2CF_3$ (IIb)
$F(M)_n-CF_2CF_3$ (IIIb)
$CF_3CF_2CF_2-O(M)_n-OCF_2CF_2CF_3$ (IVb)
$CF_3CF_2CF_2O-(M)_n-CF_2CF_3$ (Vb)
$CF_3CF_2-(M)_n-CF_2CF_3$ (VIb)
$CF_3CF_2CF_2O(M)_n-OCF_2CF_3$ (VIIb)
$CF_3CF_2CF_2O(M)_n-OCF_3$ (VIIIb)
$CF_3CF_2CF_2O(M)_n-CF_2CF_2CF_3$ (IXb)
$CF_3CF_2CF_2(M)_n-F$ (Xb)
$CF_3CF_2CF_2(M)_n-CF_2CF_3$ (XIb)
$CF_3CF_2(M)_n-OCF_3$ (XIIb)
$CF_3CF_2O(M)_n-F$ (IIa)
$CF_3O(M)_n-F$ (IXa)
$CF_3CF_2(M)_n-OCF_2CF_3$ (VIIIa)

By reducing the amount of heat evolved in the reaction medium and by promoting an effective dispersion thereof according to the previously indicated procedures, species Ia and IIb through VIb may constitute more than 90% of the obtained telomer mixture.

When $ClCF_2-CF_2OF$ is used as the telogen, the obtained telomer mixture is almost exclusively composed of the following species:

$F(M)_nF$ (Ia)
$F(M)_n-OCF_2CF_2Cl$ (IIc)
$ClCF_2-CF_2O-(M)_n-OCF_2CF_2Cl$ (IIIc)
$ClCF_2-CF_2O-(M)_n-CF_2Cl$ (IVc)
$F(M)_n-CF_2Cl$ (Vc)
$CF_2Cl(M)_n-CF_2Cl$ (VIc)
$ClCF_2CF_2O-(M)_n-OCF_3$ (VIIc)
$ClCF_2CF_2O(M)_n-OCF_2CF_3$ (VIIIc)
$CF_3CF_2O(M)_nCF_2Cl$ (IXc)
$CF_3O(M)_n-CF_2Cl$ (Xc)
$CF_3CF_2O(M)_nF$ (IIa)
$CF_3O(M)_n-F$ (IXa)

When reducing the amount of heat evolved in the reaction medium and promoting an effective dispersion thereof, according to the procedures indicated hereinbefore, species Ia and IIc through VIc may constitute more than 90% of the obtained telomer mixture.

The main advantages of the present invention can be summarized as follows:

1) inert end groups are obtained which impart high chemical and thermal stability to the telomers;

2) it is possible to vary, over a wide range, both distribution and nature of the end groups which are present in the obtained telomeric species and, to a certain extent, the telomerization degree, thus resulting in large variety of products having different physical and chemical properties, which are suited to meet various different applicative requirements;

3) it is possible to vary, over a wide range, the F/Cl ratio of the telomers obtained. Thus, it is possible to obtain products having a high F/Cl ratio and endowed with particular physical and chemical properties. Furthermore, it is possible to obtain telomers with a lower F/Cl ratio which can impart particular properties to the products such as, for example, a low compressibility.

The following examples are given merely to illustrate the present invention and are not to be construed as a limitation thereof.

## Example 1

A flow of 1.0 Nl/h of fluoroxypentafluoroethane and 3.0 Nl/h of $N_2$ was continuously bubbled through 450 g of liquid chlorotrifluoroethylene, cooled to -55 °C, in a 1-liter glass reactor equipped with a reflux condenser, a thermometer and a mechanical stirrer.
After 4 hours and 30 minutes the reaction was stopped and the unreacted CTFE (chlorotrifluoroethylene) was distilled off.
There were obtained 71 g of product; by gas-chromatographic analysis, the composition was evaluated in

terms of "n" values, whereafter it was possible to calculate a yield of about 50%, based on $CF_3$-$CF_2OF$, corresponding to about 15.8 g of telomers per liter of gaseous telogen. The product was subjected to further analysis via gas-mass spectroscopy by means of a capillary column SE-52.
Moreover, $^{19}$F-N.M.R. spectra were recorded on a BRUKER apparatus AM 300, dissolving the samples in $CDCl_3$.

From these tests it was concluded that the product consisted of telomeric species Ia to Xa, out of which species Ia to IIIa proved to constitute more than 85% of the mixture. Species IXa was present in an amount lower than 1%.
The gas-chromatographic analyses revealed that the telomers having a value of "n" from 3 to 6 represented about 90% of the crude product.

Said product was then subjected to fractionated distillation under reduced pressure. In this manner there were removed a head product consisting of CTFE residues and of non-telomeric by-products, and a tail product consisting of telomeric high-boiling products and of telomeric waxy products. The thus obtained product was a colorless oil (designated as sample 1), whose distribution in terms of "n" values, as concluded from the gas-chromatographic analysis, was as follows:

| Sample 1 | |
|---|---|
| value of "n" | area % |
| 2 + volatile matter | 27.2 |
| 3 | 16.4 |
| 4 | 36.0 |
| 5 | 18.1 |
| 6 | 0.8 |

A second distillation under reduced pressure was then carried out in order to decrease the amount of telomers having a "n" value equal to 2. Thus, sample 2 having the following distribution of the "n" values was obtained:

| Sample 2 | |
|---|---|
| value of "n" | area % |
| 2 | 2.3 |
| 3 | 25.4 |
| 4 | 45.9 |
| 5 | 22.1 |
| 6 | 4.3 |

On samples 1 and 2, the following physical and chemical properties were determined :

- density according to method ISO R/758
- viscosity according to standard ASTM D445
- pour point according to standard ASTM D99
- vapour tension according to the method of the pressure transducer as described in Journ. of Chem. and Eng. Data, vol. 13, No. 3, July 1969
- heat of combustion according to standard ASTM D240
- unsaturation according to the method illustrated in the technical handbook by Halocarbon Product Corporation
- bulk modulus by measuring the propagation rate of the supersonic waves.

The results obtained are given in the following Table 1.

TABLE 1

| Physical and chemical properties | | | |
|---|---|---|---|
| Properties | | Sample 1 | Sample 2 |
| DENSITY (g/ml) | 20 ° C<br>40 ° C | 1.8106<br>1.7722 | 1.8522<br>1.8129 |
| VISCOSITY ($mm^2/s$ = cSt) | -20 ° C<br>-18 ° C<br>-15 ° C<br>-10 ° C<br>0 ° C<br>20 ° C<br>40 ° C<br>60 ° C<br>80 ° C<br>100 ° C<br>120 ° C<br>135 ° C | -<br>10.55<br>9.34<br>7.40<br>4.95<br>2.86<br>1.99<br>1.34<br>0.99<br>0.87<br>0.71<br>0.51 | 68.8<br>-<br>-<br>31.1<br>16.4<br>6.7<br>3.4<br>2.1<br>1.4<br>1<br>0.8<br>0.67 |
| POUR POINT, ° C | | -78 | -58 |
| VAPOR TENSION AT 121 ° C, kPa (TORR) | | 137 (1030) | 36 (270) |
| COMBUSTION HEAT J/g (cal/g) | | 5903 (1410) | 6867 (1640) |
| UNSATURATION (min.) | | 140 | 415 |
| BULK MODULUS | | - | $0.93083.10^9$ Pa |

To be noticed are the particularly low values of pour point, heat of combustion and unsaturation.

Example 2

Following the procedure of example 1, 1.0 Nl/h of $CF_3$-$CF_2OF$ and 4.0 Nl/h of nitrogen were bubbled through 450 g of liquid CTFE, at -75 ° C, during 6 hours. After removal of the unreacted CTFE there were obtained 101 g of product with a yield of about 15.0 g of telomers per liter of gaseous $CF_3$-$CF_2OF$. The compounds Ia to Xa were present. About 90% of the mixture was composed of the species Ia to IIIa. The telomers having a value of "n" ranging from 3 to 6 amounted to about 93% of the total.

Example 3

Following the procedure of example 1, 2.0 Nl/h of $CF_3$-$CF_2OF$ and 4.0 Nl/h of $N_2$ were bubbled through 260 g of liquid CTFE, at -40 ° C, for 3 hours and 30 minutes. After removal of the unreacted CTFE, there were obtained 63 g of product with a yield of about 9.0 g of telomers per liter of gaseous $CF_3$-$CF_2OF$. Compounds Ia to Xa were present. The species IVa to Xa represented about 90% of the mixture.

After the unreacted CTFE was distilled off, the following distribution of the "n" values resulted:

| value of "n" | area % |
|---|---|
| 2 | not determined |
| 3 | 3.2 |
| 4 | 6.7 |
| 5 | 11.1 |
| 6 | 14.5 |
| 7 | 14.5 |
| 8 | 13.5 |
| 9 | 12.0 |
| 10 | 10.2 |
| 11 | 7.9 |
| 12 | 5.1 |
| >12 | not determined |

Example 4

Following the procedure of example 1, 1.0 Nl/h of $CF_3$-$CF_2OF$ and 3.0 Nl/h of $N_2$ were bubbled through 160 g of CTFE mixed with 160 g of $CF_2Cl$-$CF_2Cl$, at -55°C, for 6 hours.

After the unreacted CTFE and the solvent were distilled off, 71 g of product were obtained, the yield being about 11.3 g of telomers per liter of gaseous $CF_3CF_2OF$. Compounds Ia to Xa were present. About 90% of the mixture was composed of species Ia to IIIa. The telomers having a "n" value ranging from 3 to 6 amounted to 76% of the total.

Example 5

Following the procedure of example 1, 1.0 Nl/h of $CF_3$-$CF_2OF$ and 5.0 Nl/h of $N_2$ were bubbled through 200 g of CTFE mixed with 200 g of $CFCl_3$, at -75°C, for 14 hours. After the unreacted CTFE and the solvent were distilled off, there were obtained 180 g of product with a yield of about 12.9 g of telomers per liter of gaseous $CF_3$-$CF_2OF$. Compounds Ia to Xa were present. Species Ia to IIIa represented about 90% of the mixture. The telomers having a "n" value ranging from 3 to 6 corresponded to 66% of the total.

Example 6

Following the procedure of example 1, 1.0 Nl/h of $CF_3$-$CF_2$-$CF_2OF$ and 8.0 Nl/h of $N_2$ were bubbled through 260 g of CTFE, at -50°C, for 8 hours. After the unreacted CTFE was distilled off, 72 g of product were obtained. A yield of about 9.0 g of telomers per liter of gaseous $CF_3$-$CF_2$-$CF_2OF$ was calculated. The telomers IIb to XIIb, Ia, IIa, VIIIa and IXa were present. The species Ia and IIb to VIb represented about 90% of the mixture. Species IXa was present in an amount lower than 1%. The "n" values were distributed as follows:

| value of "n" | area % |
|---|---|
| 2 + volatile matter | 44.7 |
| 3 | 20.9 |
| 4 | 14.7 |
| 5 | 7.9 |
| 6 | 5.1 |
| 7 | 3.2 |
| 8 | 2.3 |
| 9 | 1.1 |
| ≧ 10 | not determined |

Example 7

Following the procedure of example 1, 1.0 Nl/h of $CF_3$-$CF_2$-$CF_2OF$ and 8.0 Nl/h of $N_2$ were bubbled through 260 g of CTFE mixed with 260 g of $CF_2Cl$-$CF_2Cl$, at -50 °C, for 5 hours and 30 minutes. After having distilled off the unreacted CTFE and the solvent, 54 g of product were obtained. A yield of about 9.7 g of telomers per liter of gaseous $CF_3$-$CF_2$-$CF_2OF$ was calculated. Compounds IIb to XIIb, Ia, IIa, IXa and VIIIa were present. More than 90% of the mixture was composed of species Ia and IIb to VIb. The telomers having "n" values from 3 to 6 amounted to about 60% of the total.

Example 8

Following the procedure of example 1, 1.0 Nl/h of $CClF_2$-$CF_2OF$ and 5.0 Nl/h of $N_2$ were bubbled through 260 g of CTFE, at -75 °C, for 7 hours and 15 minutes. After distilling-off of the unreacted CTFE, there were obtained 80 g of product with a yield of 11.0 g of telomers per liter of gaseous $CClF_2$-$CF_2OF$. Telomers IIc to Xc, Ia, IIa and IXa were present. More than 90% of the mixture consisted of species Ia and from IIc to VIc. Species IXa was present in an amount lower than 1%.

The product was then subjected to fractionated distillation at reduced pressure. Thereby there were removed a head product prevailingly consisting of CTFE residues and a tail product consisting of high-boiling products and waxy products. A colorless oil (designated as fraction 3) was obtained which had the following "n" value distribution:

| value of "n" | area % |
|---|---|
| 2 + volatile matter | 34.2 |
| 3 | 18.6 |
| 4 | 21.8 |
| 5 | 11.4 |
| 6 | 6.0 |
| 7 | 3.4 |
| ≥8 | 2.2 |

On sample 3 there were determined the same physical and chemical properties as in example 1.
The results are given in the following Table 2.

Table 2

| Physical and chemical properties of sample 3 | | |
|---|---|---|
| DENSITY , (g/ml) | 20 °C<br>40 °C | 1.8444<br>1.8053 |
| VISCOSITY , ($mm^2/s = cSt$) | -20 °C<br>-10 °C<br>0 °C<br>20 °C<br>40 °C<br>60 °C<br>80 °C<br>100 °C<br>120 °C<br>135 °C | 28.6<br>15.6<br>9.7<br>4.5<br>3<br>1.9<br>1.2<br>0.9<br>0.74<br>0.63 |
| POUR POINT, °C | | -80 |
| VAPOUR TENSION AT 121 °C, kPa (TORR) | | 45 (340) |
| COMBUSTION HEAT J/g (cal/g) | | 7578 (1810) |
| UNSATURATION (min.) | | 250 |
| BULK MODULUS | | $1,0263075.10^9$ Pa |

It is to be noticed that the pour point value, the vapor tension value and the unsaturation value are particularly low. Also the heat of combustion value is low. From a comparison of the bulk modulus value with the one of sample 2 (see example 1), a higher value can be observed, which is attributable to the presence of Cl in the end groups. Due to the high bulk modulus value, the sample is particularly suited for use in applications where a low compressibility is required.

Example 9

Following the procedure of example 1, 1.0 Nl/h of $CClF_2$-$CF_2OF$ and 5.0 Nl/h of $N_2$ were bubbled through 130 g of CTFE mixed with 160 g of $CF_2Cl$-$CF_2Cl$ at -74 °C for 9 hours. After having distilled off the unreacted CTFE and the solvent, there were obtained 59 g of product, with a yield of about 6.6 g of telomers per liter of gaseous $CClF_2$-$CF_2OF$. Compounds Ia, IXa and IIc to Xc were present. More than 90% of the mixture were composed of species Ia and IIc to VIc. About 40% of the mixture were composed of species Ia and IIc to IVc.

The telomers having a value of "n" ranging from 3 to 6 amounted to 58% of the total.

Example 10

Following the procedure of example 1, 1.0 Nl/h of n-$C_3F_7$-O-$CF(CF_3)$-$CF_2OF$ and 5.0 Nl/h of $N_2$ were bubbled through 250 g of CTFE at -74 °C for 10 hours. After having distilled off the unreacted CTFE, there were obtained 58.5 g of product.

The following telomers resulted:

$F(M)_nF$ (Ia)
$CF_3O(M)_n$-F (IXa)
$CF_3O(M)_nOCF_3$ (3)
$C_3F_7O$-$CF(CF_3)$-$(M)_n$-F (4)
$C_3F_7O$-$CF(CF_3)CF_2O(M)_n$-F (5)
$C_3F_7O$-$CF(CF_3)$-$(M)_n$-$OCF_3$ (6)
$C_3F_7O$-$CF(CF_3)$-$CF_2O(M)_n$-$OCF_3$ (7)
$CF_3$-$CF_2$-$CF_2$-$CF(CF_3)$-$CF_2$-$O(M)_n$-F (8)
$CF_3$-$CF_2$-$CF_2$-$CF(CF_3)$-$CF_2(M)_n$-$OCF_3$ (9)
$C_3F_7O$-$CF(CF_3)$-$(M)_n$-$CF(CF_3)$-$OC_3F_7$ (10)

$C_3F_7O\text{-}CF(CF_3)\text{-}(M)_n\text{-}OC_3F_6\text{-}OC_3F_7$ (11)
$C_3F_7O\text{-}C_3F_6O\text{-}(M)_n\text{-}OC_3F_6\text{-}OC_3F_7$ (12)
$CF_3\text{-}CF_2\text{-}CF_2\text{-}CF(CF_3)\text{-}CF_2\text{-}(M)_n\text{-}CF(CF_3)\text{-}OC_3F_7$ (13)
$CF_3\text{-}CF_2\text{-}CF_2\text{-}CF(CF_3)\text{-}CF_2\text{-}(M)_n\text{-}OC_3F_6\text{-}OC_3F_7$ (14)

where "n" ranges from 2 to 8.

## Claims

1. Process for preparing chlorotrifluoroethylene telomers, characterized in that chlorotrifluoroethylene is reacted, at a temperature ranging from -100° to +40°C, with a perhalofluoroxy compound of formula $R_x\text{-}CF_2OF$ wherein $R_x$ represents a perhalogenated alkyl radical, a perhaloalkylmonoether radical or a perhaloalkylpolyether radical, either linear or branched, having from 1 to 10 carbon atoms and containing fluorine atoms or fluorine and chlorine atoms, but being free of vicinal chlorine atoms and $-CCl_3$ groups.

2. The process of claim 1, characterized in that the radical $R_x$ contains from 1 to 5 carbon atoms.

3. The process of claim 1 or 2, characterized in that the radical $R_x$, when containing fluorine and chlorine atoms, is free of $-CCl_2-$ groups.

4. The process of one or more of the preceding claims, characterized in that a gaseous flow of perhalofluoroxy compound is fed to a reactor containing chlorotrifluoroethylene in the liquid state or dissolved in a solvent which is inert under the reaction conditions, e.g. a chlorofluorohydrocarbon.

5. The process of one or more of the preceding claims, characterized in that the perhalofluoroxy compound, before being fed to the reactor, is diluted with a gas which is inert under the reaction conditions, e.g. nitrogen, argon, helium or a gaseous chlorofluorohydrocarbon.

6. The process of one or more of claims 1 to 3, characterized in that the perhalofluoroxy compound is fed, in the liquid state, to a reactor containing chlorotrifluoroethylene, either in the liquid state or dissolved in a solvent which is inert under the reaction conditions, the perhalofluoroxy compound being mixed with a liquid which is inert under the reaction conditions, e .g. a chlorofluorohydrocarbon, or being carried, in the form of an aerosol, by a gas which is inert under the reaction conditions, e.g. nitrogen, argon or helium.

7. The process of one or more of the preceding claims, characterized in that the temperature ranges from -30° to -80°C.

8. Chlorotrifluoroethylene telomers having one of the following formulae A to K:

$R_x\text{-}CF_2\text{-}O\text{-}(M)_n\text{-}F$ (A)
$R_x\text{-}CF_2\text{-}O\text{-}(M)_n\text{-}O\text{-}CF_2\text{-}R_x$ (B)
$R_x\text{-}(M)_n\text{-}F$ (C)
$R_x\text{-}(M)_n\text{-}R_x$ (D)
$R_x\text{-}CF_2\text{-}O\text{-}(M)_n\text{-}R_x$ (E)
$R_x\text{-}O\text{-}(M)_n\text{-}F$ (F)
$R_x\text{-}O\text{-}(M)_n\text{-}O\text{-}CF_2\text{-}R_x$ (G)
$R_x\text{-}O\text{-}(M)_n\text{-}R_x$ (H)
$R_x\text{-}CF_2\text{-}(M)_n\text{-}F$ (I)
$R_x\text{-}CF_2\text{-}(M)_n\text{-}O\text{-}CF_2\text{-}R_x$ (J)
$R_x\text{-}CF_2\text{-}(M)_n\text{-}R_x$ (K)

in which $R_x$ is a perhalogenated alkyl radical, a perhaloalkylmonoether radical or a perhaloalkylpolyether radical, either linear or branched, having 1 to 10 carbon atoms and containing fluorine atoms or fluorine atoms and chlorine atoms, but being free of vicinal chlorine atoms and $-CCl_3$ groups; M represents $CF_2\text{-}CFCl$ and "n" ranges from 2 to 20; provided that in formula (F) $R_x$ is different from $CF_3$; in formulae (C) and (I) $R_x$ is different from perfluoroalkyl; and in formula (D) $R_x$ is different from

perhalomethyl when n ranges from 3 to 15.

**9.** Chlorotrifluoroethylene telomers having one of the following formulae L, N and O:

$R^1$-O-$(M)_n$-$OCF_2$-$R_x$ (L)
$R^1$-O-$(M)_n$-$R_x$ (N)
$R^1$-$(M)_n$-F (O)

in which $R_x$ is a perhalogenated alkyl radical, a perhaloalkylmonoether radical or a perhaloalkylpolyether radical, either linear or branched, having 1 to 10 carbon atoms and containing fluorine atoms or fluorine atoms and chlorine atoms, but being free of vicinal chlorine atoms and -$CCl_3$ groups;
M represents $CF_2$-CFCl;
$R^1$ represents a radical derived from $R_x$, in which $R_x$ having at least two carbon atoms has lost one or more carbon atoms and/or $R_x$ having at least three carbon atoms has undergone a re-arrangement, provided that in formula (O) $R^1$ is different from perfluoroalkyl; and
"n" ranges from 2 to 20.

**10.** Chlorotrifluoroethylene telomers having one of the following formulae R to V:

$R^3$-O-$(M)_n$-$OCF_2$-$R_x$ (R)
$R^3$-O-$(M)_n$-$R_x$ (S)
$R^3$-$CF_2$-O-$(M)_n$-F (T)
$R^3$-$CF_2$-O-$(M)_n$-O-$CF_2$-$R_x$ (U)
$R^3$-$CF_2$-O-$(M)_n$-$R_x$ (V)

in which $R_x$ represents a perhalogenated alkyl radical, a perhaloalkylmonoether radical or a perhaloalkyl-polyether radical, either linear or branched, having 1 to 10 carbon atoms and containing fluorine atoms and chlorine atoms, but being free of vicinal chlorine atoms and -$CCl_3$ groups;
M represents $CF_2$-CFCl;
$R^3$ is a radical derived from $R_x$, in which $R_x$ having at least two carbon atoms has lost one or more carbon atoms and/or $R_x$ having at least three carbon atoms has undergone a re-arrangement and one or more chlorine atoms of $R_x$ have been substituted by fluorine atoms; and
"n" ranges from 2 to 20.

**11.** Telomers according to any of claims 8 through 10, characterized in that the radical $R_x$ contains 1 to 5 carbon atoms.

**12.** Telomers according to any of claims 8 through 11, characterized in that the radical $R_x$, when containing fluorine atoms and chlorine atoms, is free of -$CCl_2$- groups.

**13.** Telomers according to any of claims 8 through 12, characterized in that "n" ranges from 2 to 10.

**14.** Chlorotrifluoroethylene telomer mixtures containing the species:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$R_x$-O-$(M)_n$-F (F)
$R_x$-O-$(M)_n$-O-$CF_2$-$R_x$ (G)
$R_x$-O-$(M)_n$-$R_x$ (H)
$R_x$-$CF_2$-$(M)_n$-F (I)
$R_x$-$CF_2$-$(M)_n$-O-$CF_2$-$R_x$ (J)
$R_x$-$CF_2$-$(M)_n$-$R_x$ (K)
$R^1$-O-$(M)_n$-$OCF_2$-$R_x$ (L)
$R^1$-O-$(M)_n$-$R_x$ (N)
$R^1$-$(M)_n$-F (O)

$CF_3$-O-$(M)_n$-F (P)
F-$(M)_n$-F (Q)

wherein $R_x$, M and n are defined as in any one of claims 9 and 11 to 13 and $R^1$ has the same meaning as given in claim 9 without the proviso.

**15.** Chlorotrifluoroethylene telomer mixtures containing the species:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$CF_3$-O-$(M)_n$-F (P)
F-$(M)_n$-F (Q)
$R^3$-O-$(M)_n$-O$CF_2$-$R_x$ (R)
$R^3$-O-$(M)_n R_x$ (S)
$R^3$-$CF_2$-O-$(M)_n$-F (T)
$R^3$-$CF_2$-O-$(M)_n$-O-$CF_2$-$R_x$ (U)
$R^3$-$CF_2$-O-$(M)_n$-$R_x$ (V)

in which $R_x$, M and n are defined as in any one of claims 10 to 13 and $R^3$ has the same meaning as given in claim 10.

**16.** Chlorotrifluoroethylene telomer mixtures prevailingly containing the species:

$R_x$-$CF_2$-O-$(M)_n$-F (A)
$R_x$-$CF_2$-O-$(M)_n$-O-$CF_2$-$R_x$ (B)
F-$(M)_n$-F (Q)

or prevalingly containing the species:

$R_x$-$(M)_n$-F (C)
$R_x$-$CF_2$-O-$(M)_n$-$R_x$ (E)
$R_x$-O-$(M)_n$-O-$CF_2$-$R_x$ (G)
$R_x$-$CF_2$-$(M)_n$-F (I)
$R_x$-$CF_2$-$(M)_n$-O-$CF_2$-$R_x$ (J)
$CF_3$-O-$(M)_n$-F (P)
F-$(M)_n$-F (Q)

in which $R_x$, M and "n" have the same meanings as given in claim 14.

**17.** Chlorotrifluoroethylene telomer mixtures prevailngly containing the species:

$R_x$-$(M)_n$-F (C)
$R_x$-$(M)_n$-$R_x$ (D)
$R_x$-$CF_2$-O-$(M)_n$-$R_x$ (E)
F-$(M)_n$-F (Q)
$R^3$-O-$(M)_n$-$R_x$ (S)

in which $R_x$, M, $R^3$ and "n" have the same meanings as given in claim 15.

**18.** Chlorotrifluoroethylene telomers having one of the following formulae (4) to (14):

$C_3F_7O$-CF($CF_3$)-$(M)_n$-F (4)
$C_3F_7O$-CF($CF_3$)$CF_2O(M)_n$-F (5)
$C_3F_7O$-CF($CF_3$)-$(M)_n$-O$CF_3$ (6)
$C_3F_7O$-CF($CF_3$)-$CF_2O(M)_n$-O$CF_3$ (7)

$CF_3-CF_2-CF_2-CF(CF_3)-CF_2-O(M)_n-F$ (8)
$CF_3-CF_2-CF_2-CF(CF_3)-CF_2(M)_n-OCF_3$ (9)
$C_3F_7O-CF(CF_3)-(M)_n-CF(CF_3)-OC_3F_7$ (10)
$C_3F_7O-CF(CF_3)-(M)_n-OC_3F_6-OC_3F_7$ (11)
$C_3F_7O-C_3F_6O-(M)_n-OC_3F_6-OC_3F_7$ (12)
$CF_3-CF_2-CF_2-CF(CF_3)-CF_2-(M)_n-CF(CF_3)-OC_3F_7$ (13)
$CF_3-CF_2-CF_2-CF(CF_3)-CF_2-(M)_n-OC_3F_6-OC_3F_7$ (14)

wherein M represents $CF_2$-CFCl and "n" ranges from 2 to 8.

**19.** Chlorotrifluoroethylene telomer mixtures containing the species:

$F(M)_nF$ (Ia)
$CF_3O(M)_n-F$ (IXa)
$CF_3O(M)_nOCF_3$ (3)
$C_3F_7O-CF(CF_3)-(M)_n-F$ (4)
$C_3F_7O-CF(CF_3)CF_2O(M)_n-F$ (5)
$C_3F_7O-CF(CF_3)-(M)_n-OCF_3$ (6)
$C_3F_7O-CF(CF_3)-CF_2O(M)_n-OCF_3$ (7)
$CF_3-CF_2-CF_2-CF(CF_3)-CF_2-O(M)_n-F$ (8)
$CF_3-CF_2-CF_2-CF(CF_3)-CF_2(M)_n-OCF_3$ (9)
$C_3F_7O-CF(CF_3)-(M)_n-CF(CF_3)-OC_3F_7$ (10)
$C_3F_7O-CF(CF_3)-(M)_n-OC_3F_6-OC_3F_7$ (11)
$C_3F_7O-C_3F_6O-(M)_n-OC_3F_6-OC_3F_7$ (12)
$CF_3-CF_2-CF_2-CF(CF_3)-CF_2-(M)_n-CF(CF_3)-OC_3F_7$ (13)
$CF_3-CF_2-CF_2-CF(CF_3)-CF_2-(M)_n-OC_3F_6-OC_3F_7$ (14)

wherein M and "n" have the same meanings as given in claim 18.

## Patentansprüche

**1.** Verfahren zur Herstellung von Chlortrifluorethylen-Telomeren, dadurch gekennzeichnet, daß Chlortrifluorethylen bei einer Temperatur im Bereich von -100° bis +40°C mit einer Perhalogenfluoroxyverbindung der Formel $R_x-CF_2OF$, worin $R_x$ einen perhalogenierten Alkylrest, einen Perhalogenalkylmonoetherrest oder einen Perhalogenalkylpolyetherrest, entweder linear oder verzweigt, darstellt, der 1 bis 10 Kohlenstoffatome aufweist und Fluoratome oder Fluor- und Chloratome enthält, aber frei von vicinalen Chloratomen und $-CCl_3$-Gruppen ist, umgesetzt wird.

**2.** Verfahren nach Anspruch 1, dadurch gekennzeichnet, daß der Rest $R_x$ 1 bis 5 Kohlenstoffatome enthält.

**3.** Verfahren nach Anspruch 1 oder 2, dadurch gekennzeichnet, daß der Rest $R_x$, wenn er Fluor- und Chloratome enthält, frei von $-CCl_2$-Gruppen ist.

**4.** Verfahren nach einem oder mehreren der vorangehenden Ansprüche, dadurch gekennzeichnet, daß ein gasförmiger Fluß von Perhalogenfluoroxyverbindung einem Reaktor, der Chlortrifluorethylen im flüssigen Zustand oder in einem Lösungsmittel, das unter den Reaktionsbedingungen inert ist, z.B. einem Chlorfluorkohlenwasserstoff, gelöst enthält, zugeführt wird.

**5.** Verfahren nach einem oder mehreren der vorangehenden Ansprüche, dadurch gekennzeichnet, daß die Perhalogenfluoroxyverbindung vor ihrer Einleitung in den Reaktor mit einem Gas, das unter den Reaktionsbedingungen inert ist, z.B. Stickstoff, Argon, Helium oder einem gasförmigen Chlorfluorkohlenwasserstoff, verdünnt wird.

**6.** Verfahren nach einem oder mehreren der Ansprüche 1 bis 3, dadurch gekennzeichnet, daß die Perhalogenfluoroxyverbindung im flüssigen Zustand einem Reaktor zugeführt wird, der Chlortrifluorethylen, entweder im flüssigen Zustand oder in einem Lösungsmittel, das unter den Reaktionsbedingungen inert ist, gelöst, enthält, wobei die Perhalogenfluoroxyverbindung mit einer Flüssigkeit gemischt ist, die unter den Reaktionsbedingungen inert ist, z.B. einem Chlorfluorkohlenwasserstoff, oder in Form

eines Aerosols durch ein Gas getragen wird, das unter den Reaktionsbedingungen inert ist, z.B. Stickstoff, Argon oder Helium.

**7.** Verfahren nach einem oder mehreren der vorangehenden Ansprüche, dadurch gekennzeichnet, daß die Temperatur im Bereich von -30° bis -80°C liegt.

**8.** Chlortrifluorethylen-Telomere mit einer der folgenden Formeln A bis K:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$R_x$-O-$(M)_n$-F (F)
$R_x$-O-$(M)_n$-O-$CF_2$-$R_x$ (G)
$R_x$-O-$(M)_n$-$R_x$ (H)
$R_x$-$CF_2$-$(M)_n$-F (I)
$R_x$-$CF_2$-$(M)_n$-O-$CF_2$-$R_x$ (J)
$R_x$-$CF_2$-$(M)_n$-$R_x$ (K)

in welchen $R_x$ ein perhalogenierter Alkylrest, ein Perhalogenalkylmonoetherrest oder ein Perhalogenalkylpolyetherrest, entweder linear oder verzweigt, ist, der 1 bis 10 Kohlenstoffatome aufweist und Fluoratome oder Fluor- und Chloratome enthält, aber frei von vicinalen Chloratomen und -$CCl_3$-Gruppen ist; M für $CF_2$-CFCl steht und "n" im Bereich von 2 bis 20 liegt; mit der Maßgabe, daß in Formel (F) $R_x$ von $CF_3$ verschieden ist; in den Formeln (C) und (I) $R_x$ von Perfluoralkyl verschieden ist; und in Formel (D) $R_x$ von Perhalogenmethyl verschieden ist, wenn n im Bereich von 3 bis 15 liegt.

**9.** Chlortrifluorethylen-Telomere mit einer der folgenden Formeln L, N und O:

$R^1$-O-$(M)_n$-O$CF_2$-$R_x$ (L)
$R^1$-O-$(M)_n$-$R_x$ (N)
$R^1$-$(M)_n$-F (O)

in welchen $R_x$ ein perhalogenierter Alkylrest, ein Perhalogenalkylmonoetherrest oder ein Perhalogenalkylpolyetherrest, entweder linear oder verzweigt, ist, der 1 bis 10 Kohlenstoffatome aufweist und Fluoratome oder Fluor- und Chloratome enthält, aber frei von vicinalen Chloratomen und -$CCl_3$-Gruppen ist;
M für $CF_2$-CFCl steht;
$R^1$ einen Rest darstellt, der abgeleitet ist von $R_x$, in dem $R_x$ mit wenigstens zwei Kohlenstoffatomen eines oder mehrere Kohlenstoffatome verloren hat und/oder $R_x$ mit wenigstens drei Kohlenstoffatomen sich umgelagert hat, mit der Maßgabe, daß in Formel (O) $R^1$ von Perfluoralkyl verschieden ist; und
"n" im Bereich von 2 bis 20 liegt.

**10.** Chlortrifluorethylen-Telomere mit einer der folgenden Formeln R bis V:

$R^3$-O-$(M)_n$-O$CF_2$-$R_x$ (R)
$R^3$-O-$(M)_n$-$R_x$ (S)
$R^3$-$CF_2$-O-$(M)_n$-F (T)
$R^3$-$CF_2$-O-$(M)_n$-O-$CF_2$-$R_x$ (U)
$R^3$-$CF_2$-O-$(M)_n$-$R_x$ (V)

in welchen $R_x$ einen perhalogenierten Alkylrest, einen Perhalogenalkylmonoetherrest oder einen Perhalogenalkylpolyetherrest, entweder linear oder verzweigt, darstellt, der 1 bis 10 Kohlenstoffatome aufweist und Fluoratome und Chloratome enthält, aber frei von vicinalen Chloratomen und -$CCl_3$-Gruppen ist;
M für $CF_2$-CFCl steht;
$R^3$ ein von $R_x$ abgeleiteter Rest ist, in welchem $R_x$ mit wenigstens zwei Kohlenstoffatomen eines oder mehrere Kohlenstoffatome verloren hat und/oder $R_x$ mit wenigstens drei Kohlenstoffatomen sich

umgelagert hat und eines oder mehrere Chloratome von $R_x$ durch Fluoratome ersetzt worden sind; und "n" im Bereich von 2 bis 20 liegt.

**11.** Telomere nach irgendeinem der Ansprüche 8 bis 10, dadurch gekennzeichnet, daß der Rest $R_x$ 1 bis 5 Kohlenstoffatome enthält.

**12.** Telomere nach irgendeinem der Ansprüche 8 bis 11, dadurch gekennzeichnet, daß der Rest $R_x$, wenn er Fluoratome und Chloratome enthält, frei von $-CCl_2$-Gruppen ist.

**13.** Telomere nach irgendeinem der Ansprüche 8 bis 12, dadurch gekennzeichnet, daß "n" im Bereich von 2 bis 10 liegt.

**14.** Chlortrifluorethylen-Telomermischungen, die die Spezies:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$R_x$-O-$(M)_n$-F (F)
$R_x$-O-$(M)_n$-O-$CF_2$-$R_x$ (G)
$R_x$-O-$(M)_n$-$R_x$ (H)
$R_x$-$CF_2$-$(M)_n$-F (I)
$R_x$-$CF_2$-$(M)_n$-O-$CF_2$-$R_x$ (J)
$R_x$-$CF_2$-$(M)_n$-$R_x$ (K)
$R^1$-O-$(M)_n$-$OCF_2$-$R_x$ (L)
$R^1$-O-$(M)_n$-$R_x$ (N)
$R^1$-$(M)_n$-F (O)
$CF_3$-O-$(M)_n$-F (P)
F-$(M)_n$-F (Q)

enthalten, worin $R_x$, M und n wie in irgendeinem der Ansprüche 9 und 11 bis 13 definiert sind und $R^1$ dieselbe Bedeutung wie in Anspruch 9, ohne die Maßgabe, angegeben aufweist.

**15.** Chlortrifluorethylen-Telomermischungen, die die Spezies:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$CF_3$-O-$(M)_n$-F (P)
F-$(M)_n$-F (Q)
$R^3$-O-$(M)_n$-$OCF_2$-$R_x$ (R)
$R^3$-O-$(M)_n R_x$ (S)
$R^3$-$CF_2$-O-$(M)_n$-F (T)
$R^3$-$CF_2$-O-$(M)_n$-O-$CF_2$-$R_x$ (U)
$R^3$-$CF_2$-O-$(M)_n$-$R_x$ (V)

enthalten, in denen $R_x$, M und n wie in irgendeinem der Ansprüche 10 bis 13 definiert sind und $R^3$ dieselbe Bedeutung wie in Anspruch 10 angegeben aufweist.

**16.** Chlortrifluorethylen-Telomermischungen, hauptsächlich die Spezies:

$R_x$-$CF_2$-O-$(M)_n$-F (A)
$R_x$-$CF_2$-O-$(M)_n$-O-$CF_2$-$R_x$ (B)
F-$(M)_n$-F (Q)

enthaltend oder hauptsächlich die Spezies:

$R_x$-(M)$_n$-F (C)
$R_x$-$CF_2$-O-(M)$_n$-$R_x$ (E)
$R_x$-O-(M)$_n$-O-$CF_2$-$R_x$ (G)
$R_x$-$CF_2$-(M)$_n$-F (I)
$R_x$-$CF_2$-(M)$_n$-O-$CF_2$-$R_x$ (J)
$CF_3$-O-(M)$_n$-F (P)
F-(M)$_n$-F (Q)

enthaltend, in denen $R_x$, M und "n" dieselben Bedeutungen wie in Anspruch 14 angegeben aufweisen.

**17.** Chlortrifluorethylen-Telomermischungen, die hauptsächlich die Spezies:

$R_x$-(M)$_n$-F (C)
$R_x$-(M)$_n$-$R_x$ (D)
$R_x$-$CF_2$-O-(M)$_n$-$R_x$ (E)
F-(M)$_n$-F (Q)
$R^3$-O-(M)$_n$-$R_x$ (S)

enthalten, in denen $R_x$, M, $R^3$ und "n" dieselben Bedeutungen wie in Anspruch 15 angegeben aufweisen.

**18.** Chlortrifluorethylen-Telomere mit einer der folgenden Formeln (4) bis (14):

$C_3F_7$O-CF($CF_3$)-(M)$_n$-F (4)
$C_3F_7$O-CF($CF_3$)$CF_2$O(M)$_n$-F (5)
$C_3F_7$O-CF($CF_3$)-(M)$_n$-$OCF_3$ (6)
$C_3F_7$O-CF($CF_3$)-$CF_2$O(M)$_n$-$OCF_3$ (7)
$CF_3$-$CF_2$-$CF_2$-CF($CF_3$)-$CF_2$-O(M)$_n$-F (8)
$CF_3$-$CF_2$-$CF_2$-CF($CF_3$)-$CF_2$(M)$_n$-$OCF_3$ (9)
$C_3F_7$O-CF($CF_3$)-(M)$_n$-CF($CF_3$)-$OC_3F_7$ (10)
$C_3F_7$O-CF($CF_3$)-(M)$_n$-$OC_3F_6$-$OC_3F_7$ (11)
$C_3F_7$O-$C_3F_6$O-(M)$_n$-$OC_3F_6$-$OC_3F_7$ (12)
$CF_3$-$CF_2$-$CF_2$-CF($CF_3$)-$CF_2$-(M)$_n$-CF($CF_3$)-$OC_3F_7$ (13)
$CF_3$-$CF_2$-$CF_2$-CF($CF_3$)-$CF_2$-(M)$_n$-$OC_3F_6$-$OC_3F_7$ (14)

worin M für $CF_2$-CFCl steht und "n" im Bereich von 2 bis 8 liegt.

**19.** Chlortrifluorethylen-Telomermischungen, die die Spezies:

F(M)$_n$F (Ia)
$CF_3$O(M)$_n$-F (IXa)
$CF_3$O(M)$_n$$OCF_3$ (3)
$C_3F_7$O-CF($CF_3$)-(M)$_n$-F (4)
$C_3F_7$O-CF($CF_3$)$CF_2$O(M)$_n$-F (5)
$C_3F_7$O-CF($CF_3$)-(M)$_n$-$OCF_3$ (6)
$C_3F_7$O-CF($CF_3$)-$CF_2$O(M)$_n$-$OCF_3$ (7)
$CF_3$-$CF_2$-$CF_2$-CF($CF_3$)-$CF_2$-O(M)$_n$-F (8)
$CF_3$-$CF_2$-$CF_2$-CF($CF_3$)-$CF_2$(M)$_n$-$OCF_3$ (9)
$C_3F_7$O-CF($CF_3$)-(M)$_n$-CF($CF_3$)-$OC_3F_7$ (10)
$C_3F_7$O-CF($CF_3$)-(M)$_n$-$OC_3F_6$-$OC_3F_7$ (11)
$C_3F_7$O-$C_3F_6$O-(M)$_n$-$OC_3F_6$-$OC_3F_7$ (12)
$CF_3$-$CF_2$-$CF_2$-CF($CF_3$)-$CF_2$-(M)$_n$-CF($CF_3$)-$OC_3F_7$ (13)
$CF_3$-$CF_2$-$CF_2$-CF($CF_3$)-$CF_2$-(M)$_n$-$OC_3F_6$-$OC_3F_7$ (14)

enthalten, worin M und "n" dieselben Bedeutungen wie in Anspruch 18 angegeben aufweisen.

## Revendications

1. Procédé de préparation de télomères du chlorotrifluoréthylène, caractérisé en ce qu'on fait réagir du chlorotrifluoroéthylène, à une température comprise entre -100 et +40 ° C, avec un composé perhalogénofluoroxy de formule $R_x$-$CF_2OF$, dans laquelle $R_x$ est un radical alkylperhalogéné, un radical perhalogénalkyl-monoéther ou perhalogénalkylpolyéther, linéaire ou ramifié, ayant de 1 à 10 atomes de carbone et contenant des atomes de fluor, ou des atomes de fluor et des atomes de chlore, mais ne comportant pas d'atomes de chlore et de groupes $-CCl_3$ vicinaux.

2. Le procédé selon la revendication 1, caractérisé en ce que le radical $R_x$ contient de 1 à 5 atomes de carbone.

3. Le procédé selon la revendication 1 ou 2, caractérisé en ce que le radical $R_x$, quand il contient des atomes de fluor et de chlore, ne contient pas de groupes $-CCl_2-$.

4. Le procédé selon l'une ou plusieurs des revendications précédentes, caractérisé en ce qu'on introduit un courant gazeux d'un composé perhalogénofluoroxy dans un réacteur contenant du chlorotrifluoréthylène à l'état liquide ou dissous dans un solvant inerte dans les conditions de la réaction, par exemple un hydrocarbure chlorofluoré.

5. Le procédé selon l'une ou plusieurs des revendications précédentes, caractérisé en ce que le composé perhalogénofluoroxy, avant d'être introduit dans le réacteur, est dilué avec un gaz inerte dans les conditions de la réaction, par exemple l'azote, l'argon, l'hélium ou un hydrocarbure chlorofluoré gazeux.

6. Le procédé selon l'une ou plusieurs des revendications 1 à 3, caractérisé en ce que le composé perhalogénofluoroxy est introduit à l'état liquide dans un réacteur contenant du chlorotrifluoréthylène, à l'état liquide ou dissous dans un solvant inerte dans les conditions de la réaction, le composé perhalogénofluoroxy étant mélangé à un liquide inerte dans les conditions de la réaction, par exemple un hydrocarbure chlorofluoré, ou étant porté, sous forme d'un aérosol, par un gaz inerte dans les conditions de la réaction, par exemple l'azote, l'argon ou l'hélium.

7. Le procédé selon l'une ou plusieurs des revendications précédentes, caractérisé en ce que la température est comprise entre -30 ° C et -80 ° C.

8. Télomères du chlorotrifluoréthylène ayant l'une des formules A à K suivantes:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$R_x$-O-$(M)_n$-F (F)
$R_x$-O-$(M)_n$-O-$CF_2$-$R_x$ (G)
$R_x$-O-$(M)_n$-$R_x$ (H)
$R_x$-$CF_2$-$(M)_n$-F (I)
$R_x$-$CF_2$-$(M)_n$-O-$CF_2$-$R_x$ (J)
$R_x$-$CF_2$-$(M)_n$-$R_x$ (K)

où

$R_x$ est un radical alkylperhalogéné, un radical perhalogénoalkylmonoéther ou un radical perhalogénoalkylpolyéther, linéaire ou ramifié, ayant 1 à 10 atomes de carbone et contenant des atomes de fluor ou des atomes de fluor et des atome de chlore, mais ne contenant pas d'atomes de chlore et de groupes $-CCl_3$ vicinaux;
M représente $CF_2$-CFCl, et
"n" est compris entre 2 et 20,
du moment que, dans la formule (F),
$R_x$ est différent de $CF_3$;
que, dans les formules (C) et (I):

$R_x$ est différent d'un radical perfluoralkyle; et que, dans la formule (D):

$R_x$ est différent d'un radical perhalogénométhyle quand "n" est compris entre 3 et 15.

**9.** Télomères du chlorotrifluoréthylène ayant l'une des formules suivantes (L), (N) et (O):

$R^1$-O-$(M)_n$-$OCF_2$-$R_x$ (L)
$R^1$-O-$(M)_n$-$R_x$ (N)
$R^1$-$(M)_n$-F (O)

où

$R_x$ est un radical alkylperhalogéné, un radical perhalogénoalkylmonoéther ou un radical perhalogénoalkylpolyéther, linéaire ou ramifié, ayant 1 à 10 atomes de carbone et contenant des atomes de fluor ou des atomes de fluor et des atomes de chlore, mais ne contenant pas d'atomes de chlore et de groupes $-CCl_3$ vicinaux;

M représente $CF_2$-CFCl;

$R^1$ représente un radical dérivé de $R_x$, dans lequel $R_x$, quand il possède au moins deux atomes de carbone, a perdu un ou plusieurs atomes de carbone et/ou $R_x$, quand il a au moins 3 atomes de carbone, a subit un réarrangement, du moment que, dans la formule (O), $R^1$ est différent du radical perfluoralkyle; et

"n" est compris entre 2 et 20.

**10.** Télomères du chlorotrifluoréthylène ayant l'une des formules ci-après (R) à (V):

$R^3$-O-$(M)_n$-$OCF_2$-$R_x$ (R)
$R^3$-O-$(M)_m$-$R_x$ (S)
$R^3$-$CF_2$-O-$(M)_n$-F (T)
$R^3$-$CF_2$-O-$(M)_n$-O-$CF_2$-$R_x$ (U)
$R^3$-$CF_x$-O-$(M)_n$-$R_x$ (V)

où

$R_x$ représente un radical alkylperhalogéné, un radical perhalogénoalkylmonoéther ou un radical perhalogénoalkylpolyéther, linéaire ou ramifié, ayant 1 à 10 atomes de carbone et contenant des atomes de fluor et des atomes de chlore, mais ne contenant pas d'atomes de chlore et de groupes $-CCl_3$ vicinaux;

M représente $CF_2$-CFCl;

$R^3$ est un radical dérivé de $R_x$, où $R_x$ quand il a au moins 2 atomes de carbone, a perdu un ou plusieurs atomes de carbone, et/ou $R_x$ quand il au moins 3 atomes de carbone, a subi un réarrangement et un ou plusieurs atomes de chlore de $R_x$ ont été substitués par des atomes de fluor, et

"n" est compris entre 2 et 20.

**11.** Télomères selon l'une quelconque des revendications 8 à 10, caractérisés en ce que le radical $R_x$ contient de 1 à 5 atomes de carbone.

**12.** Télomères selon l'une quelconque des revendications 8 à 11, caractérisés en ce que le radical $R_x$, quand il contient des atomes de fluor et des atomes de chlore, ne contient pas de groupes $-CCl_2-$.

**13.** Télomères selon l'une quelconque des revendications 8 à 12, caractérisés en ce que "n" est compris entre 2 et 10.

**14.** Mélanges de télomères du chlorotrifluoréthylène contenant les composés suivants:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)

$R_x$-O-$(M)_n$-F (F)
$R_x$-O-$(M)_n$-O-$CF_2$-$R_x$ (G)
$R_x$-O-$(M)_n$-$R_x$ (H)
$R_x$-$CF_2$-$(M)_n$-F (I)
$R_x$-$CF_2$-$(M)_n$-O-$CF_2$-$R_x$ (J)
$R_x$-$CF_2$-$(M)_n$-$R_x$ (K)
$R^1$-O-$(M)_n$-$OCF_2$-$R_x$ (L)
$R^1$-O-$(M)_n$-$R_x$ (N)
$R^1$-$(M)_n$-F (O)
$CF_3$-O-$(M)_n$-F (P)
F-$(M)_n$-F (Q)

où

$R_x$, M et "n" sont définis dans l'une quelconque des revendications 9 et 11 à 13, et
$R^1$ a la même signification que celle donnée dans la revendication 9, sans la condition.

**15.** Mélanges de télomères du chlorotrifluoréthylène contenant les composés suivants:

$R_x$-$CF_2$-O-$(M)_n$-F (A)
$R_x$-$CF_2$-O-$(M)_n$-O-$CF_2$-$R_x$ (B)
$R_x$-$(M)_n$-F (C)
$R_x$-$(M)_n$-$R_x$ (D)
$R_x$-$CF_2$-O-$(M)_n$-$R_x$ (E)
$CF_3$-O-$(M)_n$-F (P)
F-$(M)_n$-F (Q)
$R^3$-O-$(M)_n$-$OCF_2$-$R_x$ (R)
$R^3$-O-$(M)_n$-$R_x$ (S)
$R^3$-$CF_2$-O-$(M)_n$-F (T)
$R^3$-$CF_2$-O-$(M)_n$-O-$CF_2$-$R_x$ (U)
$R^3$-$CF_2$-O-$(M)_n$-$R_x$ (V)

où

$R_x$, M et "n" sont tel que définis dans l'une quelconque des revendications 10 à 13, et
$R^3$ a la même signification que celle donnée dans la revendication 10.

**16.** Mélanges de télomères du chlorotrifluoréthylène, contenant essentiellement les composés suivants:

$R_x$-$CF_2$-O-$(M)_n$-F (A)
$R_x$-$CF_2$-O-$(M)_n$-O-$CF_2$-$R_x$ (B)
F-$(M)_n$-F (Q)

ou contenant essentiellement les composés suivants:

$R_x$-$(M)_n$-F (C)
$R_x$-$CF_2$-O-$(M)_n$-$R_x$ (E)
$R_x$-O-$(M)_n$-O-$CF_2$-$R_x$ (G)
$R_x$-$CF_2$-$(M)_n$-F (I)
$R_x$-$CF_2$-$(M)_n$-O-$CF_2$-$R_x$ (J)
$CF_3$-O-$(M)_n$-F (P)
F-$(M)_n$-F (Q)

où

$R_x$, M et "n" ont les mêmes significations que celles données dans la revendication 14.

**17.** Mélanges de télomères du chlorotrifluoréthylène contenant essentiellement les composés suivants:

$R_x$-$(M)_n$-F (C)
$R_x$-$(M)_n$-$R_x$ (D)

$R_x-CF_2-O-(M)_n-R_x$ (E)
$F-(M)_n-F$ (Q)
$R^3-O-(M)_n-R_x$ (S)

où
$R_x$, M, $R^3$ et "n" ont les mêmes significations que celles données dans la revendication 15.

**18.** Télomères du chlorotrifluoréthylène ayant l'une des formules ci-après (4) à (14):

$C_3F_7-O-CF(CF_3)-(M)_n-F$ (4)
$C_3F_7-O-CF(CF_3)-CF_2O(M)_nF$ (5)
$C_3F_7-O-CF(CF_3)-(M)_n-OCF_3$ (6)
$C_3F_7-O-CF(CF_3)-CF_2O(M)_n-OCF_3$ (7)
$CF_3CF_2CF_2CF(CF_3)-CF_2-O(M)_n-F$ (8)
$CF_3CF_2CF_2CF(CF_3)-CF_2(M)_n-OCF_3$ (9)
$C_3F_7-O-CF(CF_3)-(M)_nCF(CF_3)OC_3F_7$ (10)
$C_3F_7-O-CF(CF_3)-(M)_nOC_3F_6OC_3F_7$ (11)
$C_3F_7-O-C_3F_6O-(M)_nOC_3F_6OC_3F_7$ (12)
$CF_3CF_2CF_2CF(CF_3)CF_2-(M)_n-CF(CF_3)-OC_3F_7$ (13)
$CF_3CF_2CF_2CF(CF_3)CF_2-(M)_n-OC_3F_6-OC_3F_7$ (14)

où
M représente $CF_2CFCl$ et
"n" est compris entre 2 et 8.

**19.** Mélange de télomères du chlorotrifluoréthylène contenant les composés suivants:

$F(M)_nF$ (Ia)
$CF_3O(M)_nF$ (IXa)
$CF_3O(M)_nOCF_3$ (3)
$C_3F_7-O-CF(CF_3)-(M)_n-F$ (4)
$C_3F_7-O-CF(CF_3)-CF_2O(M)_nF$ (5)
$C_3F_7-O-CF(CF_3)-(M)_n-OCF_3$ (6)
$C_3F_7-O-CF(CF_3)-CF_2O(M)_n-OCF_3$ (7)
$CF_3CF_2CF_2CF(CF_3)-CF_2O(M)_n-F$ (8)
$CF_3CF_2CF_2CF(CF_3)-CF_2(M)_n-OCF_3$ (9)
$C_3F_7-O-CF(CF_3)-(M)_nCF(CF_3)OC_3F_7$ (10)
$C_3F_7-O-CF(CF_3)-(M)_nC_3F_6OC_3F_7$ (11)
$C_3F_7-O-C_3F_6O-(M)_nOC_3F_6OC_3F_7$ (12)
$CF_3CF_2CF_2CF(CF_3)CF_2-(M)_n-CF(CF_3)-OC_3F_7$ (13)
$CF_3CF_2CF_2CF(CF_3)CF_2-(M)_n-OC_3F_6-OC_3F_7$ (14)

où
M et "n" ont les mêmes significations que celles données dans la revendication 18.